Europäisches Patentamt

European Patent Office

Office européen des brevets

(11) Publication number: **0 344 107**
**A1**

## (12) EUROPEAN PATENT APPLICATION

(21) Application number: **89810357.7**

(22) Date of filing: **17.05.89**

(51) Int. Cl.⁴: **A 61 F 2/04**

(30) Priority: **17.05.88 JP 120184/88**

(43) Date of publication of application:
**29.11.89 Bulletin 89/48**

(84) Designated Contracting States: **DE FR GB SE**

(71) Applicant: **ASAHI KOGAKU KOGYO KABUSHIKI KAISHA**
**36-9, Maeno-cho 2-chome Itabashi-ku**
**Tokyo (JP)**

(72) Inventor: **Ichitsuka, Takeshi**
**Asahi Logaku Kogyo K. K. 36-9 Maenocho 2-chome Itabashi-ku Tokyo (JP)**

**Ojima, Satoshi**
**Asahi Logaku Kogyo K. K. 36-9 Maenocho 2-chome Itabashi-ku Tokyo (JP)**

**Imamura, Yoshiroh**
**Asahi Logaku Kogyo K. K. 36-9 Maenocho 2-chome Itabashi-ku Tokyo (JP)**

**Ogawa, Tetsuro**
**Asahi Logaku Kogyo K. K. 36-9 Maenocho 2-chome Itabashi-ku Tokyo (JP)**

(74) Representative: **Moinas, Michel**
**Moinas & Cie 42, rue Plantamour**
**CH-1201 Genève (CH)**

(54) Artificial trachea and production process thereof.

(57) An artificial trachea of the two-layered ring structure in which an outer layer of the ring structure is composed of a porous ceramic material and an inner layer integrally bonded to said outer layer is composed of a dense ceramic material, and a production process thereof. The artificial trachea is useful as a substitute for laryngotracheal cartilage, because it has a good mechanical strength enough to act as a connective tissue in place of the cartilage, does not cause infection and inflammation, shows an excellent fixability in the surrounding soft tissue, and can be retained in vivo for a long period without suffering from troubles.

EP 0 344 107 A1

# Description

## ARTIFICIAL TRACHEA AND PRODUCTION PROCESS THEREOF

### BACKGROUND OF THE INVENTION

#### Field of the Invention

The present invention relates to an artificial trachea. More particularly, the present invention relates to an artificial trachea of the two layered ring structure and production process thereof. The artificial trachea of the present invention can be advantageously used as a connective tissue in place of the laryngotracheal cartilage.

#### Description of Related Art

Surgical operations have been widely carried out to treat constriction or partial histionic failure of the larynx and the trachea. In these operations, it is the most important problem how to repair a frame or scaffold structure of the laryngotracheal cartilage. Hitherto, attempts to overcome this problem have involved the use as a cartilage substitute of auto-cartilage, i.e., cartilage from another part of a patient's body, or synthetic materials such as silicones and ceramics. Silicones, however, have no good compatibility with the soft tissue of the patient's body and also do not exhibit a good fixability in said soft tissue.

As the ceramics for the cartilage substitute, there have been proposed to use, for example, porous or dense, apatite, zirconia, alumina and other ceramic materials. These ceramic materials are generally considered to be more useful than the silicone materials, but have some drawbacks.

Porous ceramics can exhibit an excellent fixability in the soft tissue, because the soft tissue can be gradually grown into pores of the ceramics, thereby inducing a so-called "anchoring effect". Namely, the porous ceramics can be strongly fixed in the soft tissue as a result of interlocking of the ceramics and soft tissue. However, such ceramics lack for mechanical strengths and moreover have a tendency of causing infection and inflammation. If dense ceramics are used in place of the porous ceramics there is caused a problem similar to that encountered in the use of silicone materials discussed above, i.e., problem of fixability in the soft tissue.

Recently, there have been suggest to use an artificial trachea comprising a porous tube of apatite having coated on an inner surface thereof a layer of polymethyl methacrylate resin. This artificial trachea is considered to be somewhat more useful than the above discussed prior art tracheas, because it has an excellent fixability in the soft tissue and permeation or migration of bacteria through a wall of the trachea is effectively prevented. However, because of presence of the resin layer coated onto an inner surface of the artificial trachea, the bacteria and other viruses can be adhered to and rapidly increased and grown on said inner surface.

Therefore, a need exists for an improved artificial trachea which has an excellent mechanical strength and fixability in the surrounding soft tissue of the patient's body, and has no tendency of causing migration of the bacteria and other viruses through a wall thereof and adhesion and growth thereof on a wall surface. The artificial trachea will be particularly suitable in the reconstructive surgery of the scaffold structure of the larynx and the trachea. A further need exists for an improved production process useful for the production of said artificial trachea.

#### Summary of the Invention

According to the present invention, there is provided an artificial trachea of the two-layered ring structure in which an outer layer of the ring structure is composed of a porous ceramic material and an inner layer integrally bonded to said outer layer is composed of a dense ceramic material.

Also, according to the present invention, there is provided a process for the production of an artificial trachea of the two-layered ring structure which comprises separately molding an outer ring composed of a porous ceramic material and an inner ring composed of a dense ceramic material; optionally calcinating said outer and inner rings; fitting said inner ring into said outer ring; and sintering said fitted outer and inner rings at an elevated temperature slightly higher than the sintering temperature generally applied to the conventional sintering processes for ceramics to yield an integral ring structure of said outer and inner rings upon shrink-fitting of said rings.

#### Description of the Preferred Embodiments

The artificial trachea according to the present invention, as described above, is characterized by the two-layered ring structure in which an outer layer or ring is composed of a porous ceramic material and an inner layer or ring integrally bonded to said outer layer is composed of a dense ceramic material.

The porous and dense ceramic materials used can be optionally selected from a plurality of ceramic materials with various types and pores. Selection of such ceramic materials is not limited insofar as the selected material has no toxicity in vivo, namely, it does not adversely affect on a patient's body. Useful ceramic materials include, for example, calcium phosphate ceramics, alumina ceramics, zirconia ceramics and a composite of two or more ceramics. Especially useful ceramic materials are calcium phosphate ceramic materials, among which hydroxyapatite is especially preferred, because these materials have high compatibility with the patient's body.

The porous outer layer and dense inner layer may be made from the same ceramic material or alternatively from different ceramic materials. For example, both of the outer and inner layers may be composed of calcium phosphate ceramics or zirconia ceramics. Also, the outer layer and the inner layer may be composed of hydroxyapatite and alumina, respectively.

In the artificial trachea of the present invention, the porous ceramic material constituting the outer layer or ring has preferably has a porosity of about 20 to 70 %, more preferably a porosity of about 30 to 60%. The porosity of less than 20% must be avoided, because it does not ensure a satisfactory anchoring effect. Also, the porosity of more than 70% must be avoided, because it causes reduction of the mechanical strength to a level not enough to use.

In addition to the porosity, the porous ceramic material has preferably a pore size or diameter of about 2 to 2000$\mu$m, more preferably a pore size of about 10 to 1500$\mu$m. The pore size of less than 2$\mu$m causes a drawback that the soft tissue can be grown into pores of the ceramic material with difficulty, while the pore size of more than 2000$\mu$m causes another drawback that only an insufficient anchoring effect can be obtained as a result of limitation of the number of pores contained in the ceramic material.

The size of the artificial trachea of the present invention may be widely varied depending upon a particular patient such as age and other factors. Similarly, the layer thickness of each of the outer and inner layers may be widely varied. Preferably, the outer layer as a layer thickness of about 0.5 to 20 mm, more preferably of about 1.0 to 1.5 mm, and the inner layer has a layer thickness of about 0.5 to 2.0 mm, more preferably of about 1.0 to 1.5 mm.

The artificial trachea of the present invention has many remarkable advantages. First, because an inner layer thereof is composed of dense ceramics, the artificial trachea has an enough strength to act as a connective tissue in place of the laryngotracheal cartilage. In addition, permeation of bacteria and other viruses into and adhesion and growth thereof onto the tracheas are effectively prevented and thus infection and inflammation can be completely avoided. Further, because an outer layer of the artificial trachea is composed of porous ceramics, an excellent fixability of the trachea in the surrounding soft tissue is obtained as a result of improvement of an anchoring effect which is induced based on growth of the soft tissue into pores of the outer layer of the trachea. Furthermore, using ceramics having a good bio-compatibility, the resulting artificial trachea can be in a patient's body for a notably long period without any disturbance or trouble.

The artificial trachea according to the present invention can be produced, preferably, by shrink fitting upon sintering of the outer and inner layers or rings, although the present invention is not limited to this shrink fitting.

According to the process of the present invention, an adhesion method, a slurry coating method, a sintering method or the like may be generally used in the production of the artificial tracheas. The sintering method is particularly useful, because the adhesion method has a problem of eluation of organic substances from the adhesive used, and the slurry coating method has a problem of the varied thickness of the resulting porous layer, for example.

Using the sintering method, the artificial trachea can be preferably produced by separately molding an outer ring of the porous ceramics and an inner ring of the dense ceramics, optionally calcinating said outer and inner rings, fitting said inner ring into said outer ring and simultaneously sintering said combined outer and inner rings. As a result, the outer ring and inner ring can be integrally bonded with a clamping force or effect based on shrinkage of the outer ring.

The porous ceramics used for the production of the outer ring can be prepared by using conventional methods. Typical methods useful in the production of the porous ceramics include preparing spherical ceramic particles and sintering said particles while they are contacted with each other. They also include adding a foaming agent to a slurry of ceramics and then foaming and drying the mixture. Suitable foaming agent is a substance capable of forming foams in the slurry, i.e., for example, hydrogen peroxide and egg albumen. Another useful method is to add a thermally decomposable substance to ceramic powders, mold the mixture to a desired shape and sinter the molded product. The thermally decomposable substance useful in this method includes azodicarbonamide, ammonium carbonate or the like or a vaporizable organic substance which can be removed through vaporization from the molded product during sintering thereof such as organic resin beads, organic fibers or the like. Still another useful method is to impregnate a slurry of ceramics into a combustible polymeric sponge or foam having a three-dimensional network and then sinter the impregrated and dried sponge.

After formation of the ceramic rings, they are fitted. However, for stabilization and other purposes, the rings may be optionally heated at an appropriate calcination temperature, before the fitting step. Further, as described hereinafter, the inner dense ring may be pre-sintered to ensure an integral and strong bond of the rings, if a shrinkage factor of the ceramic material of the outer ring is equal to or lower than that of the ceramic material of the inner ring.

To obtain a complete fitting of the outer and inner rings, an inner diameter of the outer ring and an outer diameter of the inner ring are preferably selected so that said inner ring can be easily inserted into an opening of said outer ring, but after sintering, an outer peripheral surface of said inner ring can integrally bond with an inner peripheral surface of said outer ring. In other words, when the inner ring is inserted into the opening of the outer ring, a small gap is generated between these rings, but such gap is completely eliminated after sintering of the rings. The size of the gap generated must be determined in each production process, because it can very to a large extent depending upon various factors such as diamerer of the opening of the rings, materials of the ceramics used and sintering temperature.

Preferably, the outer and inner rings are produced with such sizes that the inner diameter of the sintered outer ring is generally about 10 to 500$\mu$m smaller than the outer diamether of the sintered inner ring, and then the fitted rings are simulataneously sintered to form an integral ring structure. This method is particularly suited to cases in that a shrinkage factor of the porous ceramic material of the outer ring is higher than that of the dense ceramic material of the inner ring. Therefore, in other cases, i.e., cases that a shrinkage factor of the ceramic material of the outer ring is equal to or lower than that of the ceramic material of the inner ring, it is suggested to previously sinter or pre-sinter only the inner ring, before fitting thereof into the outer ring, to ensure that the inner diameter of the sintered outer ring is about 10 to 500μm smaller than the outer diameter of the sintered inner ring. After pre-sintering, the outer and inner rings are fitted and sintered in accordance with the process of the present invention.

Sintering of the fitted outer and inner rings is preferably carried out at an elevated temperature slightly higher than the sintering temperature generally applied to the conventional sintering processes for ceramics to yield an integral ring structure of the outer and inner rings upon shrink fitting of said rings. For example, sintering is preferably carried out at a temperature of about 1000 to 1250°C when both of the porous and dense ceramic materials used are calcium phosphate ceramics. Further, sintering is preferably carried out at a temperature of about 1400 to 1600°C when both of the porous and dense ceramic materials used are zirconia ceramics. Furthermore, sintering is preferably carried out at a temperature of about 1200 to 1800°C when the used porous ceramic material is hydroxyapatite and the used dense ceramic material is alumina.

The present invention will be further described by reference to the following example to which the present invention is not restricted.

Example 1;

A porous apatite ring having an inner diameter of 19.40 mm and an outer diameter of 25.30 mm was produced in accordance with a conventional hydrogen peroxide foaming method. Separately, a dense apatite ring having an inner diameter of 1.5 mm and an outer diameter of 16.30 mm was produced by a conventional dry process. The dense apatite ring was fitted into the porous apatite ring and then, the fitted rings were sintered at 1200°C. An artificial trachea of the two-layered ring structure having an outer diameter of 17 mm, inner diameter of 12 mm, layer thickness of the porous ring layer of 1.5 mm and layer thickness of the dense ring layer of 1.0 mm was thus obtained. The tests for this artificial trachea indicates that it has a good mechanical strength, it does not become an origin for infection and inflammation, it has a good fixability in the soft tissue of the human body and also it can be safely retained in vivo for a long period.

**Claims**

1. An artificial trachea of the two-layered ring structure in which an outer layer of the ring structure is composed of a porous ceramic material and an inner layer integrally bonded to said outer layer is composed of a dense ceramic material.

2. An artificial trachea according to claim 1 wherein said porous and dense ceramic materials may be the same or different and each is selected from the group consisting of calcium phosphate ceramics, alumina ceramics, zirconia ceramics and a composite thereof.

3. An artificial trachea according to claim 2 wherein said porous and dense ceramic materials are hydroxyapatite.

4. An artificial trachea according to claim 1 wherein said porous ceramic material constituting said outer layer has a porosity of 20 to 70% and a pore size of 2 to 2000μm.

5. An artificial trachea according to claim 1 wherein said outer layer of said ring structure has a layer thickness of 0.5 to 2.0 mm and said inner layer of said ring structure has a layer thickness of 0.5 to 2.0 mm.

6. An artificial trachea according to anyone of claims 1 to 5 wherein said integral bond between said outer and inner layers is produced by shrink fitting upon sintering of said layers.

7. A process for the production of an artificial trachea of the two-layered ring structure which comprises separately molding an outer ring composed of a porous ceramic material and an inner ring composed of a dense ceramic material; optionally calcinating said outer and inner rings; fitting said inner ring into said outer ring; and sintering said fitted outer and inner rings at an elevated temperature slightly higher that the sintering temperature generally applied to the conventional sintering processes for ceramics to yield an integral ring structure of said outer and inner rings upon shrink fitting of said rings.

8. A production process according to claim 7 wherein said porous and dense ceramic materials may be the same or different and each is selected from the group consisting of calcium phosphate ceramics, alumina ceramics, zirconia ceramics and a composite thereof.

9. A production process according to claim 8 wherein said porous and dense ceramic materials are hydroxyapatite.

10. A production process according to claim 7 wherein said porous ceramic material constituting said outer ring has a porosity of 20 to 70% and a pore size of 2 to 2000μm.

11. A production process according to claim 7 wherein said outer ring of said ring structure has a layer thickness of 0.5 to 2.0 mm and said inner ring of said ring structure has a layer thickness of 0.5 to 2.0 mm.

12. A production process according to claim 7 wherein an inner diameter of said outer ring and an outer diameter of said inner ring are selected so that said inner ring can be easily inserted into an opening of said outer ring, but after sintering, an outer peripheral surface of said inner ring can integrally bond with an inner peripheral surface of said outer ring.

13. A production process according to claim 12 wherein the inner diameter of said sintered outer ring is 10 to 500μm smaller than the outer diameter of said sintered inner ring.

14. A production process according to claim 13 wherein in cases that a shrinkage factor of the ceramic material of said outer ring is equal to or lower than that of the ceramic material of said inner ring, before fitting, only said inner ring is pre-sintered to ensure that the inner diameter of said isintered outer ring is 10 to 500μm smaller than the outer diameter of said sintered inner ring.

15. A production process according to claim 7 wherein sintering is carried out at a temperature of 1000 to 1250°C when both of said porous and dense ceramic materials are calcium phosphate ceramics.

16. A production process according to claim 7 wherein sintering is carried out at a temperature of 1400 to 1600°C when both of said porous and dense ceramic materials are zirconia ceramics.

17. A production process according to claim 7 wherein sintering is carried out at a temperature of 1200 to 1400°C when said porous ceramic material is hydroxyapatite and said dense ceramic material is alumina.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | TEXTIL PRAXIS INTERNATIONAL, vol. 41, no. 3, March 1986, pages 261-263, Leinfelden, Echterdingen, DE; H. PLANCK et al.: "Einsatz von textilen Strukturen in der Medizin Entwicklung einer Luftröhrenprothese" * Section 5G: "Wandstruktur unterschiedlicher Porosität"; section 6: "Entwicklung einer Verfahrenstechnik zur Prothesenherstellung"; figures 1,3,5 * | 1-17 | A 61 F 2/04 |
| Y | DE-A-3 122 345 (ROSENTHAL TECHNIK) * Claims 1,3-5; page 6, lines 15-21; page 7, lines 7-13,20-24; page 8, lines 27-28; page 9, line 27 - page 10, line 4; figures 1-2 * | 1-17 | |
| A | LIFE SUPPORT SYSTEMS, vol. 4, no. 4, October-December 1986, pages 283-287, London, GB; G.A. VOS et al.: "Tracheal reconstruction with hydroxyapatite tracheal prosthesis" * Section: "Materials and methods", page 283; "Summary" * | 1-4,7-10,15,17 | |
| A | EP-A-0 107 476 (CALCITEK) * Page 3, lines 28-33; page 4, lines 5-13; page 8, lines 31-36 * | 1-3,7-9,15,17 | |
| A | US-A-4 737 411 (G.A. GRAVES, JR. et al.) * Column 2, line 56 - column 3, line 17; column 3, lines 37-40; column 5, lines 14-28,43-48 * | 1-4,7-10,15-17 | |

TECHNICAL FIELDS SEARCHED (Int. Cl.4)

A 61 F
A 61 L

The present search report has been drawn up for all claims

| Place of search | Date of completion of the search | Examiner |
|---|---|---|
| THE HAGUE | 13-09-1989 | NICE P.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

EPO FORM 1503 03.82 (P0401)